(19) Europäisches Patentamt
European Patent Office
Office européen des brevets

(11) Veröffentlichungsnummer: **0 183 169**
**B1**

(12) **EUROPÄISCHE PATENTSCHRIFT**

(45) Veröffentlichungstag der Patentschrift: **10.05.89**

(51) Int. Cl.⁴: **C 07 D 311/30**, C 07 F 9/12, A 61 K 31/35

(21) Anmeldenummer: **85114678.7**

(22) Anmeldetag: **19.11.85**

(54) **Halogen-hydroxy-flavone.**

(30) Priorität: **26.11.84 DE 3443007**
**19.02.85 DE 3505611**

(43) Veröffentlichungstag der Anmeldung:
**04.06.86 Patentblatt 86/23**

(45) Bekanntmachung des Hinweises auf die Patenterteilung:
**10.05.89 Patentblatt 89/19**

(84) Benannte Vertragsstaaten:
**AT BE CH DE FR GB IT LI NL SE**

(56) Entgegenhaltungen:
**US-A-3 495 009**

**EUROPEAN JOURNAL OF MEDICINAL CHEMISTRY, CHIMICA THERAPEUTICA, vol. 4, no. 4, 1969, pages 298-301, Paris, FR; C. BREYSSE et al.: "Chimie des composés flavoniques. IX. Synthèse d'hydroxy ou méthoxy-4' dichloro3',5' flavones"**
**CHEMICAL ABSTRACTS, vol. 96, 1982, page 110, abstract no. 183009j, Columbus, Ohio, US; TACHIBANA, SANRO et al.: "Studies on pitch troubles caused by pulping and bleaching of tropical woods. XI. Chemical conversion of butin during bleaching processes", & MOKUZAI GAKKAISHI, 1982, 28(1), 45-58**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **Krämer, Josef, Dr., Grüner Weg 8, D-6104 Seeheim- Jugenheim (DE)**
Erfinder: **Irmscher, Klaus, Dr., Reuterallee 10, D-6100 Darmstadt- Eberstadt (DE)**
Erfinder: **Prücher, Helmut, Königsbergerstrasse 9, D-6148 Heppenheim (DE)**
Erfinder: **Hesch, Rolf- Dieter, Prof. Dr., Konstanty- Gutschow- Strasse 8, D-3000 Hannover- Kleefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1989

**Beschreibung**

Gegenstand der Erfindung sind neue Flavonderivate der Formel I

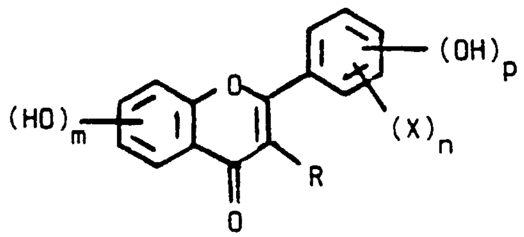

I

worin

| | |
|---|---|
| R | Alkyl mit 1 - 3 C-Atomen |
| X | F, Cl, Br oder J und |
| m, n und p | jeweils 1, 2 oder 3 bedeuten, |

sowie ihre Schwefelsäureester ("I-Sulfate") und Phosphorsäureester ("I-Phosphate") und die Salze dieser Verbindungen.

In European Journal of Medicinal Chemistry, Chimica Therapeutica, 4 (4), (1969) 298 - 301 sind ähnliche Verbindungen beschreiben, z. B. das 3',5'-Dichlor-5,7,4'-trihydroxy-flavon; jedoch finden sich dort keinerlei Angaben über pharmakologische Eigenschaften dieser Verbindungen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I, ihre Sulfate, Phosphate und die Salze dieser Verbindungen wertvolle pharmakologische Eigenschaften besitzen. So vermögen sie beispielsweise Thyroxin-5'-dejodase zu hemmen und zeigen eine erhebliche Potenz bei der Behandlung der Schilddrüsenüberfunktion; sie können selektiv die thermogene Wirkung der Schilddrüsenhormone beeinflussen. Außerdem zeigen sie antibakterielle und antitrichomonale, ferner antimutagene und daher anticancerogene Wirkungen. Weiterhin verlängern sie das Aktionspotential von Herzzellen und rufen damit eine antiarrhythmische Wirkung hervor.

Die Verbindungen der Formel I, ihre Sulfate, Phosphate und die Salze dieser Verbindungen können daher als Arzneimittelwirkstoffe in der Human- und Veterinärmedizin verwendet werden. Die Flavone I selbst sowie die I-Sulfate und die I-Phosphate dienen außerdem als Zwischenprodukte, insbesondere zur Herstellung der genannten Salze.

In der Formel I steht R vorzugsweise für Methyl, aber auch für Ethyl, Propyl oder Isopropyl. X ist bevorzugt Br, aber auch Cl oder J, ferner F. Die Parameter m und p sind vorzugsweise jeweils 1, aber auch 2 oder 3; der Parameter n ist vorzugsweise 2, ferner bevorzugt 1, aber auch 3. Es handelt sich also bei den Verbindungen der Formel I allgemein um Mono-, Di- oder Trihalogen-di-, -tri-, -tetra-, -penta- oder -hexahydroxyflavone, bevorzugt um Dihalogendihydroxy-flavone, insbesondere Dibromdihydroxyflavone, aber auch Difluordihydroxyflavone, Dichlordihydroxyflavone, Dijoddihydroxyflavone, Monobrom-monochlordihydroxyflavone, Monobrom-monojod-dihydroxyflavone, Monobrom-monofluor-dihydroxyflavone, Monochlor-monojod-dihydroxyflavone oder Monochlor-monofluor-dihydroxyflavone. Die Hydroxygruppen stehen im Benzoring des Chromonsystems bevorzugt in 6-, aber auch in 5-, 7- und/oder 8-Stellung, im Phenylring bevorzugt in 4'-, aber auch in 2'- und/oder 3'-Stellung. Im einzelnen sind besonders bevorzugt die 3',5'-Dihalogen-6,4'-, -5,4'-, -7,4'- und -8,4'-dihydroxy-flavone, z. B. die 3',5'-Dibrom-, ferner die 3',5'-Difluor-, 3',5'-Dichlor-, 3',5'-Dijod-6,4'-, -5,4'-, -7,4'- und -8,4'-dihydroxyflavone, ferner die 3'-Halogen-6,4'-, -5,4'-, -7,4'- und -8,4'-dihydroxyflavone, z. B. die 3'-Brom-, ferner die 3'-Fluor-, 3'-Chlor-, 3'-Jod-6,4'-, -5,4'-, -7,4'- und -8,4'-dihydroxyflavone der Formel I, in allen Fällen insbesondere diejenigen mit R = Methyl.

Dementsprechend sind Gegenstand der Erfindung insbesondere diejenigen Verbindungen der Formel I sowie ihre Sulfate, ihre Phosphate und die Salze dieser Verbindungen, in den mindestens einer der genannten Reste und/oder Parameter eine der vorstehend angegebenen bevorzugten Bedeutungen hat und/oder in einer der als bevorzugt angegebenen Stellungen steht. Einige bevorzugte Gruppen von Verbindungen können durch die folgenden Teilformeln worin die nicht näher bezeichneten Reste die bei der Formel I angegebenen Bedeutungen haben worin jedoch.

| | | |
|---|---|---|
| in Ia | m und p | jeweils 1 und |
| | n | 1 oder 2 bedeuten; |
| in Ib | m und p | jeweils 1 und |
| | n | 2 bedeuten; |

| | | |
|---|---|---|
| in Ic | m und p | jeweils 1, |
| | n | 2 und |
| | R | Methyl bedeuten; |
| in Id | m und p | jeweils 1 |
| | n | 2, |
| | R | Methyl und |
| | X | Br oder J bedeuten; |
| in Ie | m und p | jeweils 1 und |
| | n | 2 bedeuten, |

wobei die OH-Gruppe im Phenylring in 4'-Stellung und die beiden X-Atome in 3'- und 5'-Stellung stehen;

| | | |
|---|---|---|
| in If | m und p | jeweils 1, |
| | n | 2, |
| | R | Methyl und |
| | X | Br oder J bedeuten, |

wobei die OH-Gruppe im Phenylring in 4'-Stellung und die beiden X-Atome in 3'- und 5'-Stellung stehen.

Die Verbindungen der Formel I werden im übrigen nach an sich bekannten Methoden hergestellt, wie sie in der Literatur (z. B. in Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Der Ausdruck "dehydrierende Mittel" ist nach der Erfindung in weiterem Sinne zu verstehen. Geeignet sind beispielsweise Halogene wie Chlor, Brom oder Jod, N-Haloamide, Selendioxid, Wasserstoffperoxid, Dehydrierungskatalysatoren wie Palladium, vorzugsweise in Gegenwart eines Wasserstoffacceptors, halogenierte Chinone wie Chloranil und 2,3-Dichlor-5,6-dicyanchinon, Pyridiniumbromid-perbromid und andere Stoffe, die aktives Halogen erzeugen. Die Dehydrierung kann einstufig oder auch in mehreren Stufen erfolgen.

Man kann zur Herstellung der Flavonderivate der Formel I die Flavanone II selbst mit dehydrierenden Mitteln behandeln. Es ist aber auch möglich, die Flavanone II nicht in Substanz zu isolieren, sondern nur in situ zu erzeugen.

Beispielsweise kann man ein Keton (Chalkon) der Formel IV

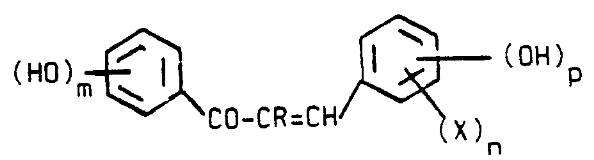

IV

worin

R, X, m, n und p die in Anspruch 1 angegebene Bedeutung haben und wobei phenolische Hydroxygruppen auch in geschützter Form vorliegen können,

anstelle der Flavanone II in die Reaktion einsetzen. Dabei bilden sich intermediär die Flavanone II.

Die Chalkone bzw. Flavanone werden vorzugsweise erhalten durch Kondensation eines entsprechenden Alkyl-phenylketons mit einem entsprechenden Halogen-hydroxy-aldehyd oder auch durch Friedel-Crafts- oder Hoesch-Synthese aus einem entsprechenden mehrwertigen Phenol und einem reaktionsfähigen Derivat einer Halogen-hydroxy-zimtsäure.

Es ist ferner möglich, so zu arbeiten, daß man auch das Keton IV nicht isoliert, sondern nur in situ erzeugt. Beispielsweise kann man das Alkyl-phenyl-keton mit dem Halogen-hydroxy-aldehyd zur Reaktion bringen und auf das Reaktionsgemisch eines der genannten Dehydrierungsmittel einwirken lassen.

Zur Dehydrierung kann man beispielsweise die Flavanone II mit Halogenen, vorzugsweise mit Chlor oder Brom, behandeln und anschließend Halogenwasserstoff abspalten. Geht man von den Ketonen IV aus, so entstehen dabei intermediär die Chalkondihalogenide, die unter Einwirkung von basischen Mitteln, vorzugsweise methanolischem oder ethanolischem Natrium- oder Kaliumhydroxid, 2 Mol Halogenwasserstoff verlieren und unter gleichzeitiger Cyclisierung in Flavone übergehen. Bei dieser Umsetzung kann die phenolische OH-Gruppe in 2-Stellung des Ketons IV auch in geschützter Form, beispielsweise als Ester- oder als Tetrahydropyranylethergruppe vorliegen; die Hydroxygruppe kann durch Einwirkung von Säuren oder Alkalien in Freiheit gesetzt werden. Geht man von einem Flavanon II aus, so kann die Reaktion über die Stufe

des 3-Haloflavanons geleitet werden. Beispielsweise gelingt es, in das Flavanonderivat II durch Bromierung unter Lichteinwirkung ein Halogenatom in 2- oder 3-Stellung einzuführen. Die Dehydrohalogenierung des erhaltenen 2- oder 3-Haloflavanons erfolgt z. B. mit alkoholischem Alkali, z. B. wässerig-ethanolischem Kaliumhydroxid oder auch durch Einwirkung tertiärer Amine, wie Kollidin, Lutidin, Pyridin, Picolin, ferner mit Lithiumchlorid oder -bromid und Lithiumcarbonat in Dimethylformamid, vorzugsweise bei Temperaturen zwischen etwa 0 und etwa 100°.

Eine andere Dehydrierungsmethode besteht in der Umsetzung des Ketons IV bzw. Flavanons II mit Selendioxid, vorzugsweise in der Hitze in einem hochsiedenden Lösungsmittel wie Xylol, Amylalkohol oder Acetanhydrid. Arbeitet man in Acetanhydrid, so ist ein intermediärer Schutz von phenolischen Hydroxygruppen nicht notwendig. Andernfalls gelingt die Reaktion besser mit geschützten, z. B. veresterten Hydroxygruppen.

Ein weiteres Dehydrierungsmittel ist Wasserstoffperoxid in alkalischer Lösung. Geht man von einem Chalkon aus, so läuft die Reaktion über das Epoxid und das 3-Hydroxyflavanon. Man arbeitet vorzugsweise in wässeriger, wässerig-alkoholischer oder alkoholischer, beispielsweise methanolischer Lösung und bei Raumtemperatur, zu Beginn der Reaktion auch unter Kühlung.

Eine weitere Methode ist die Dehydrierung der Flavanone mit Palladium in Gegenwart eines Wasserstoffacceptors. Als Acceptoren verwendet man vorzugsweise ungesättigte Säurederivate wie Zimtsäure, Maleinsäureanhydrid oder ähnliche Verbindungen. Vorzugsweise arbeitet man dabei in Gegenwart eines inerten Lösungsmittels vie Wasser und etwa zwischen 50 und 120°.

Man kann zu den Flavonen der Formel I auch gelangen durch Cyclisierung eines Ketons der Formel III, worin Z die in Anspruch 1 unter (a) angegebene Bedeutung hat, das auch in der entsprechenden Diketoform vorliegen kann. Diese Cyclisierung kann beispielsweise erfolgen durch mehrstündiges Erhitzen mit Glycerin, vorzugsweise unter Stickstoff, Einwirkung von Säuren, wie konzentrierter Salzsäure, Bromwasserstoffsäure, Jodwasserstoffsäure, Schwefelsäure, Phosphorsäure, Essigsäure, Ameisensäure oder Gemischen derselben, vorzugsweise bei Temperaturen zwischen etwa 20 und 150°.

Die als Ausgangsstoffe verwendeten Ketone brauchen bei der Reaktion nicht isoliert zu werden. Sie sind z. B. erhältlich durch Umsetzung eines entsprechenden Polyhydroxyacylophenons mit einem Halogen-hydroxybenzoesäureester unter den üblichen Bedingungen einer Esterkondensation.

Es ist auch möglich, zu den Flavonen I zu gelangen, indem man eine Carbonsäure der Formel III, worin Z die in Anspruch 1 unter (b) angegebene Bedeutung hat, cyclisiert. Das kann z. B. erfolgen durch Einwirkung von Acetylchlorid/Schwefelsäure, Phosphoroxychlorid oder Polyphosphorsäure oder auch durch Umwandlung in das entsprechende Säurehalogenid, beispielsweise mit Thionylchlorid und anschließende intramolekulare Friedel-Crafts-Acylierung, beispielsweise in Gegenwart von Aluminiumchlorid. Auch entsprechende Ester können unter hydrolysierenden Bedingungen zur Cyclisierung verwendet werden.

Die genannten Carbonsäuren sind beispielsweise erhältlich durch Umsetzung eines entsprechenden Phenols mit einem α-(Halogenhydroxy-benzoyl)-fettsäureester (Simonis-Reaktion); dabei braucht die Carbonsäure nicht isoliert zu werden.

Verbindungen der Formel I sind weiterhin erhältlich, indem man eine Verbindung, die der Formel I entspricht, aber an Stelle eines oder mehrerer H-Atome eine oder mehrere solvolysierbare oder hydrogenolysierbare Gruppe(n) enthält, mit einem solvolysierenden oder hydrogenolysierenden Mittel behandelt.

Als solvolysierbare, vorzugsweise hydrolysierbare Gruppen kommen z. B. in Frage Ester- oder Ethergruppen wie O-Alkanoyl, z. B. Acetoxy; O-Aroyl, z. B. Benzoyloxy; O-Alkyl, z. B. Methoxy oder Ethoxy; Tetrahydropyranyloxy. Beispielsweise kann man veresterte oder veretherte Hydroxygruppen in basischem, neutralem oder saurem Medium hydrolysieren. O-Alkylgruppen können z. B. durch Erhitzen mit HBr odr HJ in Essigsäure gespalten werden. Line Spaltung von Benzyloxygruppen gelingt auch hydrogenolytisch, z. B. mit Wasserstoff an einem Edelmetallkatalysator wie Pd-Kohle bei Temperaturen zwischen 0 und 100° und Drucken zwischen etwa 1 und 100 bar in einem inerten Lösungsmittel.

Die I-Sulfate und I-Phosphate sind vorzugsweise erhältlich durch Verestern einer Verbindung der Formel I mit Schwefelsäure, Phosphorsaure oder einem zur Veresterung geeigneten Derivat dieser Säuren. Je nach den Bedingungen und der Zahl der in I vorhandenen OH-Gruppen kann man I-Mono-, -Di-, -Tri-, -Tetra-, -Penta- oder -Hexa-sulfate oder -phosphate erhalten.

Für die Umsetzung geeignete Säurederivate sind neben der freien Schwefel- und Phosphorsäure vor allem Sulfaminsäure, Chlorsulfonsäure, Schwefeltrioxid und dessen Addukte mit Dioxan, Pyridin, Dimethyl- oder Diethylanilin oder anderen tertiären Basen; Pyrophosphorsäure, Polyphosphorsäure, Phosphorpentoxid, Phosphoroxychlorid, Monochlorphosphorsäure (Gemisch aus Orthophosphorsäure und Phosphoroxychlorid), Phosphorsäuremonobenzylester, Phosphorsäure-dibenzylester-chlorid, Phosphorsaure-mono-(2-cyanethylester), Phosphorsäuredimorpholid-chlorid.

Die Veresterung des Halogen-hydroxy-flavons der Formel I erfolgt in Abwesenheit oder in Gegenwart eines zusätzlichen Lösungsmittels. Als Lösungsmittel eignen sich vorzugsweise organische Basen wie Pyridin, Triethylamin, Chinolin, Dimethylanilin, Diethylanilin, falls bei der Umsetzung eine Säure, z. B. Chlorwasserstoff, abgespalten wird. Andernfalls oder zusätzlich können inerte organische Lösungsmittel verwendet werden, wie z. B. Diethyl- oder Diisopropylether, Tetrahydrofuran, Dioxan, Chloroform, Methylenchlorid, Trichlorethylen, Dimethylformamid, Dimethylsulfoxid, Benzol, Toluol, Xylol, Tetralin, Acetonitril. Ferner können Gemische der vorstehenden Basen und/oder Lösungsmittel verwendet werden. Es ist auch möglich, die Reaktion in einem Überschuß des Veresterungsmittels ablaufen zu lassen. Die Reaktionstemperaturen liegen zwischen -80 und

+200°, vorzugsweise zwischen -10 und +100°.

Ganz allgemein können zur Herstellung der I-Sulfate, der I-Phosphate und ihrer Salze die Verfahren verwendet werden, die in Houben-Weyl, l.c., Band VI/2, Seiten 452 - 464, und Band XII/2, Seiten 143 - 210 (1964), beschrieben sind.

Bei der Herstellung der Verbindungen der Formel I, ihrer Sulfate oder ihrer Phosphate können Zwischenprodukte entstehen, die Schutzgruppen enthalten. Diese können hydrolytisch oder hydrogenolytisch entfernt werden. Insbesondere können geschützte Hydroxygruppen durch alkalische oder durch vorsichtige saure Hydrolyse in Freiheit gesetzt werden. Solche geschützten Hydroxygruppen können der Flavanoidkomponente, vorzugsweise aber der Schwefelsäure- oder der Phosphorsäure-Komponente der Veresterungsreaktion entstammen. Hat man beispielsweise mit Phosphorsäure-mono-(2-cyanethylester), Phosphorsäurediphenylester-chlorid oder Phosphorsäure-dimorpholidchlorid verestert, so können die erhaltenen Phosphorsäure-di- bzw. -triester bzw. -monoesterdiamide mit z. B. Alkali- oder Ammoniumhydroxid-Lösungen, basischen oder sauren Ionenaustauschern zu den gewünschten Flavanoidphosphorsäureestern gespalten werden. Hydrogenolytisch können Schutzgruppen, vorzugsweise Benzylgruppen in Phosphorsäureestern, beispielsweise durch katalytische Hydrierung abgespalten werden, vorzugsweise unter milden Bedingungen, etwa mit einem Palladiumkatalysator wie Palladium auf Kohle, Calciumcarbonat- oder Strontiumcarbonat sowie bei Raumtemperatur und Normaldruck, wobei man die Hydrierung nach Aufnahme der berechneten Wasserstoffmenge zweckmäßig abbricht.

Die Halogen-hydroxy-flavone der Formel I sowie ihre Schwefelsäure- und Phosphorsäureester können durch Behandeln mit einer Base in die zugehorigen Salze umgewandelt werden. Als Salze kommen die Phenolate der Halogen-hydroxy-flavone I, vor allem aber die Salze der I-Sulfate und I-Phosphate in Betracht.

Bevorzugt sind physiologisch unbedenkliche Salze. Die Salze werden in der Regel bei Raumtemperatur hergestellt, wobei man als Lösungmittel vornehmlich Wasser, Alkohole wie Methanol oder Ethanol, Gemische von Wasser mit Alkoholen oder die zur Salzbildung herangezogenen Basen verwendet. Als Basen sind vorzugsweise geeignet die Hydroxide, Carbonate oder Alkoholate der Alkali- und Erdalkalimetalle sowie die entsprechenden Ammoniumverbindungen, vorzugsweise Natrium-, Kalium-, Calcium- oder Magnesiumhydroxid, Natrium-, Kalium-, Calcium- oder Magnesiumcarbonat, Natrium-, Kalium-, Calcium- oder Magnesiumbicarbonat, Natrium-, Kalium-, Calcium- oder Magnesiummethylat, -ethylat, -isopropylat oder -tert.-butylat, ferner Ammoniumhydroxid, -carbonat oder -bicarbonat, sowie substituierte Ammoniumhydroxide, -carbonate oder -bicarbonate.

Gegenstand der Erfindung ist ferner die Verwendung der Verbindungen der Formel I, ihrer Schwefelsäure- und Phosphorsäureester sowei der Salze dieser Verbindungen zur Herstellung pharmazeutischer Zubereitungen, insbesondere auf nicht-chemischem Wege. Hierbei können sie zusammen mit mindestens einem festen, flüssigen oder halbflüssigen Träger- oder Hilfsstoff und gegebenenfalls in Kombination mit einem oder mehreren veiteren Wirkstoff(en) in eine geeignete Dosierungsform gebracht werden.

Gegenstand der Erfindung sind ferner pharmazeutische Zubereitungen, enthaltend mindestens eine Verbindung der Formel I und/oder einen ihrer Schwefelsäureester und/oder einen ihrer Phosphorsäureester und/oder ein physiologisch unbedenkliches Salz einer dieser Verbindungen.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale) oder parenterale Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, Suspensionen, Emulsionen oder Implantate. Die neuen Verbindungen können auch lyophilisert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten.

Dir Verbindungen der Formel I, ihre Schwefelsäureester, Phosphorsäureester und/oder die physiologisch unbedenklichen Salze dieser Verbindungen können bei der Bekämpfung von Krankheiten, insbesondere der Schilddrüsenüberfunktion, verwendet werden. Dabei werden die erfindungsgemäßen Substanzen in der Regel in Analogie zu bekannten im Handel befindlichen Thyreostatika, z. B. Thiamazol, verabreicht, vorzugsweise in Dosierungen zwischen etwa 1 und etwa 500 mg, insbesondere zwischen 5 und 100 mg pro Dosierungseinheit. Die tägliche Dosierung liegt vorzugsweise zwischen etwa 0,015 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind die Temperaturen in °C angegeben. "Übliche Aufarbeitung" bedeutet: man gibt Wasser und Dichlormethan hinzu, trennt ab, trocknet die organische Phase über Natriumsulfat, dampft ein und reinigt durch Kristallisation und/oder Chromatographie an Kieselgel.

**Beispiel 1**

Eine Lösung von 4,12 g 3',5'-Dibrom-6,4'-dihydroxy-3-methylflavanon (F. 200 - 202°; erhältlich durch Reaktion von 2,5-Dihydroxypropiophenon mit 3,5-Dibrom-4-hydroxybenzaldehyd in siedendem Ethanol in Gegenwart won Piperidin) in 55 ml Dioxan wird unter Rühren und Bestrahlung (500 Watt Agaphot-Lampe) tropfenweise mit 1,6 g Brom in 6 ml Chloroform versetzt. Man rührt noch 30 Minuten unter Kühlung und Bestrahlung, arbeitet wie üblich auf und löst das erhaltehe rohe 3,3,5'-Tribrom-6,4'-dihydroxy-3-methyl-flavanon in 100 ml Ethanol. Es wird mit 15 ml 10 %-iger wässeriger Kalilauge versetzt, 5 Minuten gekocht, nach dem Abkühlen auf Eis gegossen und wie üblich aufgearbeitet. Man erhält 3',5'-Dibrom-6,4'-dihydroxy-3-methylflavon ("D"), F. 264 - 265°.

Analog erhält man aus den entsprechenden Flavanonen durch Bromierung die entsprechenden 3-Bromflavanone und daraus durch Dehydrobromierung:

3',5'-Dibrom-5,4'-dihydroxy-3-methylflavon, F. 238 - 242°
3',5'-Dibrom-7,4'-dihydroxy-3-methylflavon, F. 3300 (Zers.)
3',5'-Dibrom-8,4'-dihydroxy-3-methylflavon, F. 315 - 316°
3',5'-Dibrom-6,4'-dihydroxy-3-ethylflavon
3',5'-Dibrom-6,4'-dihydroxy-3-propylflavon
3',5'-Dibrom-6,4'-dihydroxy-3-isopropylflavon
3'-Fluor-6,4'-dihydroxy-3-methylflavon
3'-Chlor-6,4'-dihydroxy-3-methylflavon
3'-Brom-6,4'-dihydroxy-3-methylflavon
3'-Jod-6,4'-dihydroxy-3-methylflavon
3',5'-Difluor-6,4'-dihydroxy-3-methylflavon
3',5'-Dichlor-6,4'-dihydroxy-3-methylflavon
3',5'-Dijod-6,4'-dihydroxy-3-methylflavon.

**Beispiel 2**

Ein Gemisch von 1 g 3-(3,5-Dibrom-4-hydroxyphenyl)-1-(2,5-dihydroxyphenyl)-2-methyl-2-propen-1-on (erhältlich aus 2,5-Dihydroxypropiophenon und 3,5-Dibrom-4-hydroxybenzaldehyd), 1 g $SeO_2$ und 30 ml Isoamylalkohol wird 5 Stunden gekocht. Man filtriert, dampft das Filtrat ein, arbeitet wie üblich auf und erhält "D", F. 264 - 265°.

Analog erhält man

3',5'-Dijod-6,4'-dihydroxy-3-methylflavon F. 252 - 256°
3',5'-Dijod-5,4'-dihydroxy-3-methylflavon
3',5'-Dijod-7,4'-dihydroxy-3-methylflavon
3',5'-Dijod-8,4'-dihydroxy-3-methylflavon
3',5'-Dijod-6,4'-dihydroxy-3-ethylflavon
3',5'-Dijod-6,4'-dihydroxy-3-propylflavon
3',5'-Dijod-6,4'-dihydroxy-3-isopropylflavon
3',5'-Dichlor-6,4'-dihydroxy-3-methylflavon
3',5'-Dichlor-5,4'-dihydroxy-3-methylflavon
3',5'-Dichlor-7,4'-dihydroxy-3-methylflavon
3',5'-Dichlor-8,4'-dihydroxy-3-methylflavon
3',5'-Dichlor-6,4'-dihydroxy-3-ethylflavon
3',5'-Dichlor-6,4'-dihydroxy-3-propylflavon
3',5'-Dichlor-6,4'-dihydroxy-3-isopropylflavon
3',5'-Difluor-6,4'-dihydroxy-3-methylflavon
3',5'-Difluor-5,4'-dihydroxy-3-methylflavon
3',5'-Difluor-7,4'-dihydroxy-3-methylflavon
3',5'-Difluor-8,4'-dihydroxy-3-methylflavon
3',5'-Difluor-6,4'-dihydroxy-3-ethylflavon
3',5'-Difluor-6,4'-dihydroxy-3-propylflavon
3',5'-Difluor-6 4'-dihydroxy-3-isopropylflavon.

**Beispiel 3**

Ein Gemisch von 1 g 3-(3,5-Dibrom-4-hydroxyphenyl)-1-(2,5-dihydroxy-phenyl)-2-methyl-propan-1,3-dion [erhältlich durch Reaktion von 2,5-Dihydroxypropiophenon mit 3,5-Dibrom-4-hydroxybenzoylchlorid zu 2-(3,5-Dibrom-4-hydroxybenzoyloxy)-5-hydroxy-propiophenon und Behandeln mit KoH/Pyridin], 6 ml Essigsäure und 0,2 ml $H_2SO_4$ wird 1 Stunde auf 90° erhitzt, auf Eis gegossen und wie üblich aufgearbeitet. Man erhält "D", F. 264 - 265°.

Analog erhält man

3'-Brom-6,4'-dihydroxy-3-methylflavon
3'-Brom-5,4'-dihydroxy-3-methylflavon
3'-Brom-7,4'-dihydroxy-3-methylflavon
3'-Brom-8,4'-dihydroxy-3-methylflavon
3'-Brom-6,4'-dihydroxy-3-ethylflavon
3'-Brom-6,4'-dihydroxy-3-propylflavon
3'-Brom-6 4'-dihydroxy-3-isopropylflavon
3'-Fluor-6,4'-dihydroxy-3-methylflavon
3'-Fluor-5,4'-dihydroxy-3-methylflavon
3'-Fluor-7,4'-dihydroxy-3-methylflavon
3'-Fluor-8,4'-dihydroxy-3-methylflavon
3'-Fluor-6,4'-dihydroxy-3-ethylflavon
3'-Fluor-6,4'-dihydroxy-3-propylflavon
3'-Fluor-6,4'-dihydroxy-3-isopropylflavon
3'-Chlor-6,4'-dihydroxy-3-methylflavon
3'-Chlor-5,4'-dihydroxy-3-methylflavon
3'-Chlor-7,4'-dihydroxy-3-methylflavon
3'-Chlor-8,4'-dihydroxy-3-methylflavon
3'-Chlor-6,4'-dihydroxy-3-ethylflavon
3'-Chlor-6,4'-dihydroxy-3-propylflavon
3'-Chlor-6,4'-dihydroxy-3-isopropylflavon
3'-Jod-6,4'-dihydroxy-3-methylflavon
3'-Jod-5,4'-dihydroxy-3-methylflavon
3'-Jod-7,4'-dihydroxy-3-methylflavon
3'-Jod-8,4'-dihydroxy-3-methylflavon
3'-Jod-6,4'-dihydroxy-3-ethylflavon
3'-Jod-6,4'-dihydroxy-3-propylflavon
3'-Jod-6,4'-dihydroxy-3-isopropylflavon.

**Beispiel 4**

Eine Lösung von 1 g 3',5'-Dibrom-6-hydroxy-4'-methoxy-3-methylflavon (erhältlich durch Kondensation von 2,5-Dihydroxypropiophenon mit 3,5-Dibrom-4-methoxybenzoesäureanhydrid) in 10 ml Essigsäure und 5 ml 67 %-iger Jodwasserstoffsäure wird 1 Stunde gekocht. Nach dem Abkühlen fällt "D" aus; F. 264 - 265°.

Analog sind durch Etherspaltung der entsprechenden 4'-Methoxyverbindungen die übrigen in Beispiel 1 - 3 genannten Verbindungen erhältlich.

**Beispiel 5**

Ein Gemisch von 4,1 g "D", 3 g Sulfaminsäure und 35 ml Pyridin wird 1 Stunde bei 90° gerührt, abgekühlt und filtriert. Das Filtrat wird mit 36 ml 12 %-iger Natronlauge geschüttelt. Man trennt die - obere - Pyridinschicht ab, wäscht mehrfach mit Ether, nimmt das erhaltene Öl in Methanol auf, behandelt mit Aktivkohle, dampft ein und erhält das Dinatriumsalz des 3',5'-Dibrom-6,4'-dihydroxy-3-methylflavon-6-sulfats, F. >325°.

Analog sind erhältlich:

3',5'-Dibrom-5,4'-dihydroxy-3-methylflavon-5-sulfat-di-Na-Salz
3',5'-Dibrom-7,4'-dihydroxy-3-methylflavon-7-sulfat-di-Na-Salz
3',5'-Dibrom-8,4'-dihydroxy-3-methylflavon-8-sulfat-di-Na-Salz.

**Beispiel 6**

Zu einer Lösung von 10 ml $POCl_3$ in 100 ml Pyridin wird bei 0° innerhalb 10 Minuten eine Lösung von 4,1 g "D" in 50 ml Pyridin gegeben. Nach 15-stündigem Stehen rührt man in Eis/Salzsäure, erhitzt 1 Stunden auf 95°, kühlt ab, extrahiert mit Ethylacetat, wäscht den Extrakt mit verdünnter Salzsäure und trocknet über $Na_2SO_4$. Durch Konzentrieren des Extrakts erhält man 3',5'-Dibrom-6,4'-dihydroxy-3-methylflavon-6-phosphat, F. 258 - 260°.

Analog sind erhältlich:

3',5'-Dibrom-5,4'-dihydroxy-3-methylflavon-5-phosphat
3',5'-Dibrom-7,4'-dihydroxy-3-methylflavon-7-phosphat
3',5'-Dibrom-8,4'-dihydroxy-3-methylflavon-8-phosphat.

Die nachstehenden Beispiele betreffen pharmazeutische Zubereitungen, die Verbindungen der Formel I oder ihre physiologisch unbedenklichen Salze enthalten:

**Beispiel A:** Tabletten

Ein Gemisch von 1 kg "D", 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

**Beispiel B:** Dragees

Analog Beispiel A werden Tabletten gepreßt, die anschließend in üblicher Weise mit einem Überzug aus Saccharose, Kartoffelstärke, Talk, Tragant und Farbstoff überzogen werden.

**Beispiel C:** Kapseln

Man füllt 10 kg "D" in üblicher Weise in Hartgelatinekapseln, so daß jede Kapsel 20 mg Wirkstoff enthält.

**Beispiel D:** Ampullen

Eine Lösung von 1 kg 3',5'-Dibrom-6,4'-dihydroxy-3-methylflavon-6-sulfat-Na-salz in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.
Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

## Patentansprüche

1. Halogen-hydroxy-flavone der Formel I

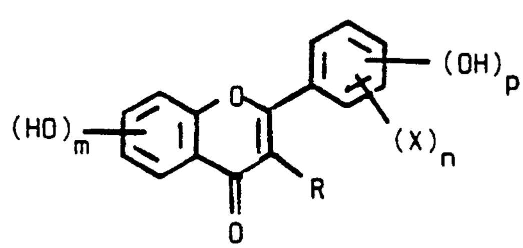

I

worin

R Alkyl mit 1 - 3 C-Atomen,

X F, Cl, Br oder J und
m, n und p jeweils 1, 2 oder 3 bedeuten, sowie deren Schwefelsäure- und Phosphorsäureester und die Salze dieser Verbindungen.

2. 3',5'-Dibrom-6,4'-dihydroxy-3-methyl-flavon.

3. Verfahren zur Herstellung von Verbindungen der Formel I sowie von deren Schwefelsäure- und Phosphorsäureestern und den Salzen dieser Verbindungen, dadurch gekennzeichnet, daß man eine Verbindung der Formel II

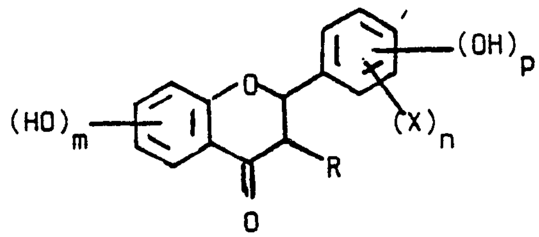

II

worin

R, X, m, n und p die in Anspruch 1 angegebenen Bedeutungen haben

mit einem dehydrierenden Mittel behandelt oder daß man eine Verbindung der Formel III

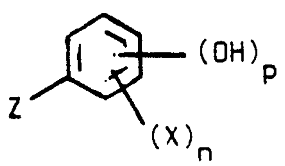

III

worin

Z 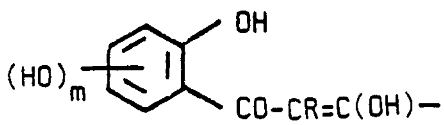 oder 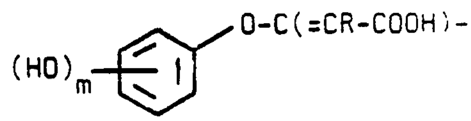

(a) (b)

bedeutet und

R, X, m, n und p die in Anspruch 1 angegebenen Bedeutungen haben

oder ein reaktionsfähiges Derivat einer solchen Verbindung cyclisiert

oder daß man eine Verbindung, die der Formel (entspricht, aber an Stelle eines oder mehrerer H-Atome eine oder mehrere solvolysierbare oder hydrogenolysierbare Gruppe(n) enthält, mit einem solvolysierenden oder hydrogenolysierenden Mittel behandelt

und/oder daß man eine Verbindung der Formel I mit Schwefelsäure, Phosphorsäure oder einem zur Veresterung geeigneten Derivat dieser Säuren verestert

und/oder daß man eine Verbindung der Formel I oder einen ihrer Schwefelsäure- oder Phosphorsäureester durch Behandeln mit einer Base in ein Salz überführt.

4. Verfahren zur Herstellung pharmazeutischer Zubereitungen, dadurch gekennzeichnet, daß man eine Verbindung der Formel I, einen ihrer Schwefelsäure- oder Phosphorsäureester und/oder ein physiologisch unbedenkliches Salz einer dieser Verbindungen zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I, einem ihrer Schwefelsäure- oder Phosphorsäureester und/oder einem physiologisch unbedenklichen Salz einer dieser Verbindungen.

6. Verbindungen der Formel I, ihre Schwefelsäure- oder Phosphorsaureester und die Salze dieser Verbindungen zur Bekämpfung von Krankheiten.

7. Verwendung einer Verbindung der Formel I, ihrer Schwefelsäure- oder Phosphorsäureester und/oder der

Salze dieser Verbindungen zur Herstellung von Arzneimitteln.

## Claims

1. Halogenohydroxyflavones of the formula

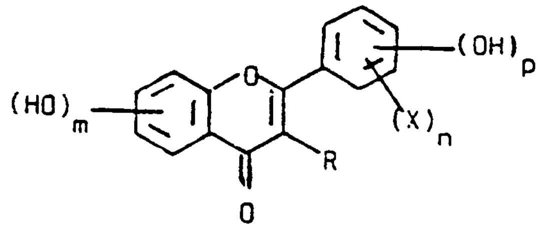
I

in which

R is alkyl having 1 - 3 C atoms,
X is F, Cl, Br or I, and
m, n and p are each 1, 2 or 3,

and their esters with sulfuric acid and phosphoric acid, and the salts of these compounds.

2. 3',5'-Dibromo-6,4'-dihydroxy-3-methylflavone.

3. Process for the preparation of compounds of the formula I, and of their esters with sulfuric acid and phosphoric acid, and the salts of these compounds, characterised in that a compound of the formula II

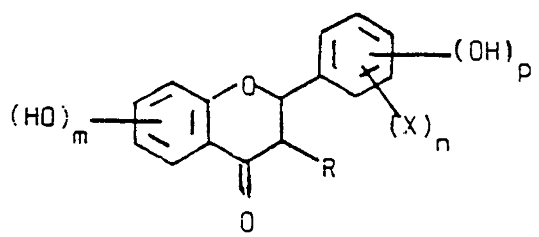
II

in which

R, X, m, n and p have the meanings indicated in Claim 1,

is treated with a dehydrogenating agent, or in that a compound of the formula

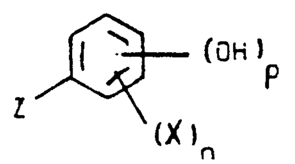
III

in which

Z is 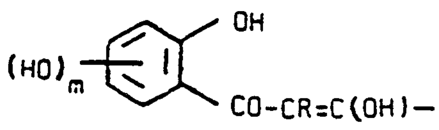 or 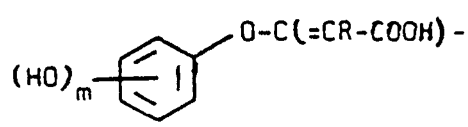

(a) (b)

and

R, X, m, n and p have the meanings indicated in Claim 1,

or a reactive derivative of a compound of this type, is cyclised,

or in that a compound which corresponds to the formula I but in place of one or more H atoms contains one or more solvolysable or hydrogenolysable group(s) is treated with a solvolysing or hydrogenolysing agent,

and/or in that a compound of the formula I is esterified with sulfuric acid, phosphoric acid or a derivative of these acids which is suitable for esterification,

and/or in that a compound of the formula I, or one of its esters with sulfuric acid or phosphoric acid, is converted into a salt by treatment with a base.

4. Process for the preparation of pharmaceutical formullations, characterised in that a compound of the formula I, one of its esters with sulfuric acid or phosphoric acid, and/or a physiologically acceptable salt of one of these compounds, is converted into a suitable dosage form together with at least one solid, liquid or semiliquid vehicle or auxiliary and, where appropriate, combined with one or more other active compound(s).

5. Pharmaceutical formulation characterised by containing at least one compound of the formula I, one of its esters with sulfuric acid or phosphoric acid, and/or a physiologically acceptable salt of one of these compounds.

6. Compounds of the formula I, their esters with sulfuric acid or phosphoric acid, and the salts of these compounds, for controlling diseases.

7. Use of a compound of the formula I, its ester with sulfuric acid or phosphoric acid, and/or the salts of these compounds, for the manufacture of drugs.

**Revendications**

1. Halogéno-hydroxy-flavones de fomrule I

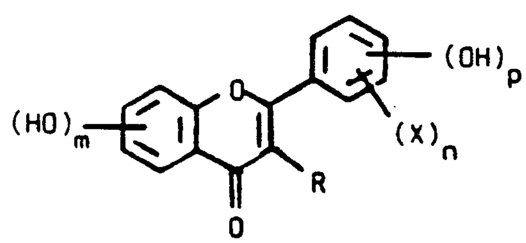 I

où

R représente un alkyle avec 1 à 3 atomes de C,
X F, Cl, Br ou I et
m, n, et p 1, 2 ou 3,

ainsi que leurs esters de l'acide sulfurique et de l'acide phosphorique et les sels de ces composés.

2. 3',5'-dibromo-6,4'-dihydroxy-3-méthyl-flavone.

3. Procédé de préparation des composés de formule I ainsi que de leurs esters de l'acide sulfurique et de l'acide phosphorique et des sels de ces composés, caractérisé en ce que l'on traite un composé de formule II

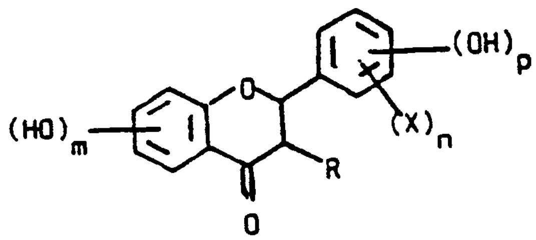 II

où

R, X, m, n et p ont les significations indiquées dans la revendication 1

avec un agent déshydrogénant ou en ce que l'on cyclise un composé de formule III

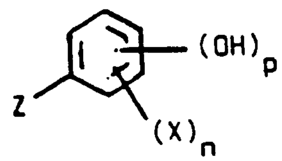 III

ou Z représente

Z 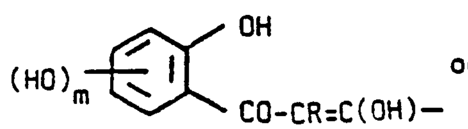 oder 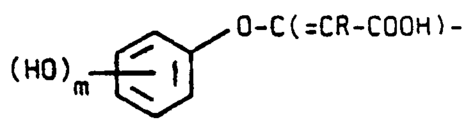

(a) (b)

R, X, m, n et p ont les significations indiquées dans la revendication 1,
ou un dérivé réactif d'un tel composé,
ou en ce que l'on traite un composé correspondant à la formule I, mais contenant à la place d'un ou de plusieurs atomes d'H un ou plusieurs groupe(s) solvolysable(s) ou hydrogénolysable(s), avec un agent solvolysant ou hydrogénolysant,
et/ou en ce que l'on estérifie un composé de formule I avec l'acide sulfurique, l'acide phosphorique ou un dérivé de ces acides approprié pour l'estérification,
et/ou en ce que l'on transforme un composé de formule I ou un de ses esters de l'acide sulfurique ou de l'acide phosphorique en un sel par traitement avec une base.

4. Procédé de préparation de compositions pharmaceutiques, caractérisé en ce que l'on associe un composé de formule I, un de ses esters de l'acide sulfurique ou phosphorique et/ou un sel physiologiquement acceptable d'un de ces composés avec au moins un support ou un adjuvant solide, liquide ou semi-liquide et éventuellement en combinaison avec une ou plusieurs autre(s) substance(s) active(s) dans une forme de dosage appropriée.

5. Composition pharmaceutique, caractérisée en ce qu'elle contient au moins un composé de formule I, un de ses esters de l'acide sulfurique ou de l'acide phosphorique et/ou un sel physiologiquement acceptable de l'un de ces composés.

6. Composés de formule I, leurs esters de l'acide sulfurique ou de l'acide phosphorique et les sels de ces composés pour lutter contre des maladies.

7. Utilisation d'un composé de formule I, de ses esters de l'acide sulfurique ou de l'acide phosphorique et/ou des sels de ces composés pour la préparation de médicaments.